# DEMANDE DE BREVET EUROPEEN

(11) **EP 2 281 538 A1**
(43) Date de publication de la demande: **09.02.2011**
(21) Numéro de dépôt: 10305867.3
(22) Date de dépôt: 06.08.2010
(51) Int. Cl.: A61F 13/505, A61F 13/514

(54) **Couche lavable**

(30) Priorité: 07.08.2009 FR 0955580
(71) Demandeur: Guillaubez, Arnaud, 01100 Oyonnax (FR)
(72) Inventeur: Guillaubez, Rachel, F-01100, OYONNAX (FR)
(74) Mandataire: Rhein, Alain

(57) **Abrégé**

L'invention concerne une couche (1) lavable comportant, depuis l'extérieur (A) vers une surface intérieure (2) en contact avec le corps du porteur (3):
- une enveloppe extérieure (4) imperméable, comportant une surface intérieure et une face extérieure en contact avec l'extérieur (B);
- un insert absorbant de recueil (5), apte à retenir du fluide;
- un voile intérieur de transfert (6), apte à venir en contact avec le porteur, et apte à transférer le fluide organique vers l'insert absorbant de recueil (5).

Elle comporte, entre l'enveloppe extérieure (4) et l'insert (5), un voile intermédiaire (12) réalisé dans un matériau perméable et qui présente, du côté de l'insert absorbant (5), une surface déperlante conçue apte à faire rouler le fluide vers l'insert absorbant (5).

## Description

L'invention concerne une couche lavable comportant, depuis l'extérieur vers une surface intérieure conçue apte à être en contact avec le corps d'un porteur:
- une enveloppe extérieure, comportant une face extérieure et une surface intérieure, ladite face extérieure étant conçue apte à être en contact avec un milieu extérieur, et ladite enveloppe extérieure étant imperméable aux liquides;
- un insert absorbant de recueil, conçu apte à retenir du fluide organique évacué dans la couche par son porteur;
- un voile intérieur de transfert, conçu apte à venir en contact avec le porteur de ladite couche, et conçu apte à transférer ledit fluide organique vers ledit insert absorbant de recueil.

L'invention concerne le domaine de la protection corporelle, et en particulier celui des couches, aussi bien pour bébés et jeunes enfants, que pour adultes, ou encore pour animaux. Dans ce domaine, on distingue, d'une part les couches jetables à usage unique, coûteuses et génératrices d'un important volume de déchets, et donc d'un coût de retraitement conséquent, et d'autre part les couches lavables, qui sont lavées à chaque change, et réutilisables pendant une très longue durée. L'invention, si elle est aussi utilisable pour des couches jetables, est principalement conçue pour le domaine des couches lavables.

Abstraction faite des couches faites d'une pièce de tissu, d'usage général jusqu'au milieu du vingtième siècle, une couche lavable comporte généralement, depuis l'extérieur vers le corps du porteur :
- une enveloppe extérieure, en contact avec les vêtements ou le lit du porteur, et imperméable aux liquides;
- un insert absorbant de recueil, conçu apte à retenir les fluides organiques évacués dans la couche par son porteur;
- un voile intérieur de transfert, en contact avec la peau du porteur, conçu apte à transférer ces fluides organiques vers cet insert absorbant de recueil.

L'enveloppe extérieure imperméable est composée, dans les réalisations connues, d'un tissu enduit, l'enduction assurant alors l'étanchéité, ou bien d'un polyester du côté extérieur, contrecollé vers le côté intérieur avec une membrane qui assure l'étanchéité, cette membrane étant constituée de polyuréthane ou similaire.

L'insert absorbant est, selon le cas, disposé dans une poche à l'intérieur de la couche, ou attaché à l'intérieur de la couche par des boutons-pression, du velours-crochet, ou similaire.

Le voile intérieur de transfert est généralement choisi en tissu micropolaire ou en suédine. Ce choix permet un bon transfert d'humidité vers l'insert absorbant, le transfert est réalisé rapidement, et le voile intérieur choisi dans un tel matériau redevient à peu près sec après le passage du liquide, ce qui préserve la peau du porteur.

Les systèmes existants présentent le défaut d'un risque élevé de transfert de liquide vers l'extérieur de la couche, surtout lors du vieillissement du tissu enduit qui en constitue l'enveloppe extérieure. La réutilisation de la couche est peu durable.

Par ailleurs, l'enveloppe extérieure imperméable présente des inconvénients. En effet, elle déborde latéralement l'insert ou le voile intérieur, en sorte de venir partiellement au contact de la peau du porteur, ce qui génère, du fait de son caractère imperméable, un certain confinement qui est souvent la cause d'érythèmes fessiers.

Par ailleurs encore, on peut constater à l'usage que les inserts absorbants, après utilisation, comportent des zones moins mouillées que d'autres, voire sèches, conséquence d'une mauvaise diffusion du fluide dans la couche, ce qui peut générer des fuites, et donc nécessiter un remplacement plus fréquent de l'insert.

Enfin, les élastiques d'entrejambe sont disposés entre l'enveloppe extérieure imperméable et la poche perméable ou le voile intermédiaire perméable, en sorte que ces élastiques sont exposés aux agressions acides des fluides et/ou des matières organiques, avec pour conséquence une moindre longévité.

L'invention se propose de pallier les divers problèmes de l'état de la technique en proposant un dispositif sécurisant pour son porteur, mais aussi pour l'entourage de ce dernier, en assurant une étanchéité parfaite, durable dans le temps et résistant aux lavages de la couche. La couche selon l'invention est, encore, conçue évolutive, pour répondre à un besoin d'économie de place de rangement ainsi que de coût d'investissement, et permet à l'utilisateur de disposer d'un modèle unique accompagnant la croissance des jeunes enfants.

A cet effet, l'invention concerne une couche lavable comportant, depuis l'extérieur vers une surface intérieure conçue apte à être en contact avec le corps d'un porteur:
- une enveloppe extérieure, comportant une face extérieure et une surface intérieure, ladite face extérieure étant conçue apte à être en contact avec un milieu extérieur, et ladite enveloppe extérieure étant imperméable aux liquides;
- un insert absorbant de recueil, conçu apte à retenir du fluide organique évacué dans la couche par son porteur;
- un voile intérieur de transfert, conçu apte à venir en contact avec le porteur de ladite couche, et conçu apte à transférer ledit fluide organique vers ledit insert absorbant de recueil.
caractérisée par le fait que ladite couche lavable comporte, entre ladite enveloppe extérieure et ledit insert, un voile intermédiaire réalisé dans un matériau perméable et qui présente, du côté dudit insert absorbant, une surface déperlante conçue apte à faire rouler ledit fluide vers ledit insert absorbant.

Le voile intermédiaire perméable et déperlant permet d'apporter des solutions aux inconvénients des couches lavables existantes.

La perméabilité du voile intermédiaire lui permet de conserver sa respirabilité, et permet de réduire les problèmes d'érythèmes fessiers.

L'enveloppe extérieure imperméable est, de ce fait, plus rigide et moins extensible que le voile intermédiaire déperlant, aussi, ce dernier déborde vers l'extérieur au niveau des élastiques de l'entrejambe pour lui permettre de former un gousset augmentant ainsi l'étanchéité de la couche, et également rendre glissantes contre la peau les parties qui portent les élastiques, pour éviter des risques de cisaillement.

La déperlance du voile intermédiaire permet de guider les fluides, et ainsi d'optimiser la capacité d'absorption de l'insert absorbant.

La déperlance du voile intermédiaire rend ce dernier hydrophobe, en sorte qu'il n'est pas souillé par les fluides organiques, et que la couche peut être réutilisée plusieurs fois sans avoir à être lavée.

Enfin, les élastiques de l'entrejambe sont disposés entre le tissu extérieur imperméable et le voile intermédiaire déperlant, c'est-à-dire entre deux tissus hydrophobes, le protège des agressions acides des fluides ou matières organiques, lui donnant une plus grande résistance dans le temps.

Selon une autre caractéristique, ledit voile intermédiaire déperlant est extensible et comporte de 15 à 25% d'élasthanne.

Selon une autre caractéristique, ledit voile intermédiaire déperlant est extensible et comporte du « Lycra » ou est constitué entièrement de « Lycra »

D'autres caractéristiques et avantages de l'invention ressortiront de la description détaillée qui va suivre des modes de réalisation non limitatifs de l'invention, en référence aux figures annexées dans lesquelles :
- la figure 1 représente, de façon schématisée et en perspective, une couche selon l'invention, le milieu dit intérieur étant représenté vers le haut de la figure, et un milieu dit extérieur étant représenté vers le bas de la figure;
- la figure 2 représente de façon schématisée, partielle et en coupe transversale, une section de la couche de la figure 1;
- la figure 3 représente, de façon schématisée, en vue de dessus depuis l'extérieur, la couche de la figure 1 mise à plat ;
- la figure 4 représente, de façon schématisée et en vue de dessous depuis l'intérieur, la couche de la figure 1 mise à plat ;
- la figure 5 représente, de façon schématisée, partielle, et en coupe, la couche de la figure 1 enveloppant un porteur.

L'invention concerne le domaine de la protection corporelle, et en particulier celui des couches, aussi bien pour bébés et jeunes enfants, que pour adultes, ou encore pour animaux.

L'invention, si elle est aussi utilisable pour des couches jetables, est principalement conçue pour le domaine des couches lavables.

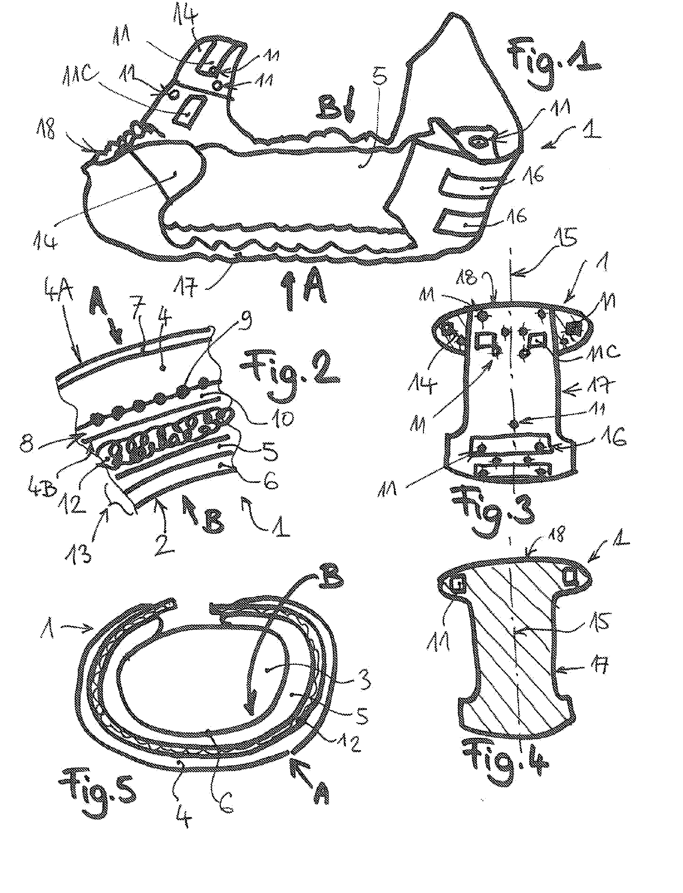

L'invention concerne une couche 1 lavable qui comporte, tel que visible sur les figures 1 et 2, depuis l'extérieur A vers l'intérieur B, et plus précisément vers une surface intérieure 2 conçue apte à être en contact avec le corps d'un porteur 3:
- une enveloppe extérieure 4, en contact avec un milieu extérieur, et imperméable aux liquides;
- un insert absorbant de recueil 5, conçu apte à retenir du fluide organique évacué dans la couche par son porteur 3;
- un voile intérieur de transfert 6 ou une bourrette de soie, conçu apte à venir en contact avec le porteur 3 de la couche 1, et conçu apte à transférer du fluide organique évacué par un porteur 3 vers l'insert absorbant de recueil 5.

L'enveloppe extérieure 4, imperméable aux liquides, comporte au moins, sur une première face extérieure 4A vers le milieu extérieur A, un tissu 7. Elle comporte, au moins sur une seconde face intérieure 4B, tourné vers le milieu intérieur B, un moyen d'étanchéité 8.

Le tissu 7 est destiné à venir en contact avec le milieu extérieur A, les vêtements ou le lit du porteur 3.

Le moyen d'étanchéité 8 a pour fonction de prévenir tout transfert d'humidité, ou plus généralement, de fluides ou matières organiques rejetés par le porteur 3, entre l'insert absorbant de recueil 5 et le milieu extérieur A

L'insert absorbant de recueil 5, par exemple constitué de molleton de bambou ou similaire, peut être disposé dans une poche à l'intérieur de la couche 1, ou attaché à l'intérieur de la couche par des moyens de fixation rapide 11 tels que boutons-pression, du velours-crochet, ou similaire.

Le voile intérieur de transfert 6 est de préférence choisi en tissu micropolaire ou en suédine, ou encore en textile à base de bambou, ou, de façon innovante et très efficace, en bourrette de soie. Ce choix permet un bon transfert d'humidité vers l'insert absorbant, le transfert est réalisé rapidement, et le voile intérieur de transfert 6 choisi dans un tel matériau redevient à peu près sec après le passage du liquide, ce qui préserve la peau du porteur 3.

Selon l'invention, l'enveloppe extérieure 4 est imperméable sur sa face intérieure 4B, et la couche lavable 1 comporte, entre l'enveloppe extérieure 4 et l'insert absorbant de recueil 5, un voile intermédiaire perméable 12 comportant une surface intérieure déperlante 12B, qui est conçue apte à faire rouler le fluide, évacué dans la couche 1 par le porteur 3, vers l'insert absorbant de recueil 5.

De façon avantageuse, l'enveloppe extérieure 4 comporte sur sa face extérieure 4A un tissu 7 qui est contrecollé, grâce à un moyen de contrecollage 9 tels que des points de colle ou un film de colle, avec une membrane étanche 10 sur sa face intérieure 4B. Cette membrane étanche est constituée d'au moins une couche mince de matière plastique telle que du polyuréthane, ou de caoutchouc, ou similaire. Cette membrane étanche 10 vient en contact avec le voile intermédiaire déperlant 12.

De façon avantageuse, le voile intermédiaire déperlant 12 est extensible et comporte de 15 à 25% d'élasthanne.

Dans une réalisation préférée, le voile intermédiaire déperlant 12 est extensible et comporte du « Lycra » ou est constitué entièrement de « Lycra ».

Dans un mode de réalisation préféré de l'invention, le voile intérieur de transfert 6 est fixé de façon permanente à l'insert absorbant de recueil 5, constituant un ensemble amovible 13 conçu apte à être fixé sur la surface intérieure déperlante 12B du voile intermédiaire déperlant 12, par des moyens de fixation rapide 11, de préférence des boutons-pression ou similaires.

De façon avantageuse, le voile intermédaire 12 est déperlant, c'est-à-dire qu'il fait rouler le liquide à sa surface, sans toutefois être imperméable. On comprend à ce propos que la membrane étanche 9 est la seule à assurer la fonction d'étanchéité. Le voile intermédiaire déperlant 12 est constitué d'un textile dont au moins une des surfaces qui constitue la surface intérieure déperlante 12B, est rendue déperlante. Pour ce faire, une telle surface peut être rendue déperlante par imprégnation d'un bain comportant de la saponine, ou encore par imprégnation d'un bain comportant au moins une résine fluorée. Naturellement ces traitements rendant le voile intermédiaire 12 déperlant ne sont pas limitatifs, ils sont retenus car ils ne présentent pas de risque sanitaire pour le porteur 3 de la couche 1 selon l'invention.

La couche 1 doit envelopper une partie du corps du porteur 3, tel que visible sur la figure 5. La figure 2 montre que, selon l'invention, le porteur 3 est en contact avec, ou bien l'insert absorbant de recueil 5 au travers du voile intérieur de transfert 6, ou bien avec le voile intermédiaire déperlant 12, mais jamais avec l'enveloppe extérieure 4.

Pour faciliter la pose, et surtout le bon maintien en position enveloppée de la couche 1 sur le porteur 3, le voile intermédiaire 12 comporte au moins une ailette 14 extensible, saillant de l'enveloppe extérieure 4, et munie de moyens de fixation rapide 11 conçus aptes à coopérer avec des moyens de fixation rapide complémentaires 11C disposés sur la surface extérieure 4A de l'enveloppe extérieure 4. De façon préférée, la couche 1 comporte un axe de symétrie longitudinal 15, et, en symétrie par rapport à cet axe 15, au moins deux ailettes 14.

La couche lavable 1 selon l'invention est, dans une version préférée, conçue évolutive, avec différents moyens de fixation rapide complémentaires 11C, disposés sur la surface extérieure 4A. Ces moyens 11C sont alors répartis sur des lignes sensiblement parallèles les unes aux autres et sensiblement perpendiculaires à l'axe de symétrie 15. Tel que visible sur les figures 3 et 4, chaque ailette 14 comporte sur la face interne 12B un moyen de fixation 11, par exemple un velours ou du crochet, et l'enveloppe extérieure 4 comporte sur sa face extérieure 4A des moyens de fixation complémentaires 11C constitués par exemple d'une ou plusieurs bandes 16, respectivement de crochet ou de velours pour constituer avec le moyen de fixation 11 une fixation ferme de type velours-crochet. On comprend que l'espacement des bandes 16 entre elles autorise l'utilisation d'une même couche 1 pour des porteurs de taille très variable. Ainsi, dans le cas d'exécution pour bébés et jeunes enfants, une même couche 1 permet son utilisation par des enfants d'une masse de 3 à 17 kg, par le positionnement judicieux de différentes bandes 16.

Une couche 1 est en général destinée à envelopper le bas-ventre, la combinaison, entre d'une part des bandes 16 et d'autre part des ailettes 14 ou bien de moyens de fixation 11 disposés à d'autres endroits de la couche, permet d'assurer, le réglage du tour de ventre grâce en particulier à l'extensibilité des ailettes 14, mais aussi le réglage du tour de cuisse. Ces réglages peuvent ainsi être effectués de façon totalement indépendante, et permettent d'assurer le confort du porteur 3, en même temps qu'un bon maintien et une bonne étanchéité périphérique.

Dans une version particulière de l'invention, tel que visible sur la figure 1, chaque ailette peut être conjuguée avec un rabat comportant des moyens de fixation complémentaires 11C aux moyens de fixation 11 que comporte l'ailette 14, de façon à ce que, lors du lavage, on puisse fixer ce rabat sur cette ailette, et ainsi éviter que le velours-crochet de cette dernière ne vienne en prise avec d'autres velours-crochets de la même couche 1 ou encore d'autres couches, lors du lavage. A ce propos, les moyens de fixation 11 dont est équipée l'ailette 14 sont de préférence des moyens de type crochet. Ainsi, les moyens de fixation complémentaire 11C tels que les bandes 16, qui, elles, sont plutôt réalisées en velours, afin, précisément, de limiter les interférences lors du lavage.

La couche lavable 1 selon l'invention est donc conçue pour une durée d'utilisation importante, de plusieurs mois voire plusieurs années pour un même porteur 3. Il est donc important que tous ses éléments présentent une bonne longévité dans le temps. A cet effet, la membrane étanche 10 est traitée résistante aux rayons ultra-violets, respirante, et résistante à l'humidité. Dans le même but, les qualités de déperlance du voile intérieur déperlant, qui peuvent s'altérer après de nombreux lavages, peuvent être facilement ravivées par l'utilisateur, par imprégnation d'un bain comportant de la saponine, ou encore par imprégnation d'un bain comportant au moins une résine fluorée.

De façon avantageuse, les moyens de fixation rapide 11 et des moyens de fixation rapide complémentaires 11C sont réalisés sous la forme de velours-crochet dont chaque élément est renforcé par une pièce de renfort constituée de la même matière que le tissu 7 et fixée, de préférence cousue, sous ce dernier. Des pièces de renfort sont aussi fixées sous les éléments des boutons-pression quand la couche en comporte.

Pour faciliter l'enveloppement, la couche 1 comporte de préférence des élastiques latéraux 17, symétriques par rapport à l'axe de symétrie 15, conçus pour enserrer les cuisses du porteur 3, et aussi de préférence au moins un élastique frontal 18 sensiblement perpendiculaire à l'axe 15.

De façon préférée, ces élastiques latéraux 17 sont cousus sur le côté extérieur de la couche 1 après l'assemblage de cette dernière par couture entre elles de l'enveloppe extérieure 4 et du voile intermédiaire déperlant 12. On comprend en effet, que, mises à part la ou les ailettes 14, l'enveloppe extérieure 4 et le voile intermédiaire 12 ont de préférence une forme identique, ce qui permet d'utiliser le même patron ou le même programme de découpe. Tel que visible sur la figure 4, du côté intérieur B de la couche 1, la totalité de la surface intérieure est constituée par le voile intermédiaire déperlant 12, sur lequel est préférentiellement fixé l'insert absorbant de recueil 5. Dans l'exemple d'exécution ci-dessus, l'insert 5 est en forme de livret et a une longueur de 34 cm environ, et est fixé au voile intermédiaire déperlant 12 par des moyens de fixation rapide 11 constitués de préférence par des boutons pression, qui sont préférés du côté intérieur de la couche, car n'engendrant pas de frottement désagréable ou blessure dans le cas de leur ouverture non intentionnelle. Les moyens de fixation sous forme de velours-crochet sont, quant à eux, préférés pour le côté extérieur 4, en raison de leur facilité de pose et de leur grande résistance à l'arrachement et au cisaillement. Cette disposition des élastiques de maintien permet que la couche 1, une fois retournée, couvre l'élastique. La même disposition peut être appliquée également à tout élastique frontal 18.

Le maintien par élastique assure ainsi que, quand la couche est refermée et enveloppe son porteur 3, seul, en dehors de l'insert 5 et du voile intérieur de transfert 6, le voile intermédiaire déperlant 12 peut être en contact avec la peau du porteur 3, et en aucun cas le tissu extérieur 7.

Le tissu extérieur 7 de l'enveloppe extérieure 4 peut être, grâce à l'assemblage de cette dernière par contrecollage avec la membrane imperméable 10, choisi dans toute nature de textile, contrairement à l'art antérieur du tissu enduit qui n'autorisait pas un large choix de matières. Il est désormais possible d'utiliser, pour le tissu 7, des textiles tels que toile jean, à base de coton ou/et de chanvre ou/et de bambou, ou tout textile à la mode du moment. Naturellement, le tissu extérieur 7 peut aussi être choisi de même nature que le voile intermédiaire déperlant 12, en particulier le cas où celui-ci comporte ou est constitué de « Lycra » confère une douceur et une esthétique agréables pour le porteur 3 comme pour l'utilisateur qui effectue la pose de la couche 1.

## Revendications

1. Couche (1) lavable comportant, depuis l'extérieur (A) vers une surface intérieure (2) conçue apte à être en contact avec le corps d'un porteur (3):
- une enveloppe extérieure (4), comportant une face extérieure (4A) et une surface intérieure (4B), ladite face extérieure (4A) étant conçue apte à être en contact avec un milieu extérieur (B), et ladite enveloppe extérieure (4) étant imperméable aux liquides;
- un insert absorbant de recueil (5), conçu apte à retenir du fluide organique évacué dans la couche (1) par son porteur (3);
- un voile intérieur de transfert (6), conçu apte à venir en contact avec le porteur (3) de ladite couche (3), et conçu apte à transférer ledit fluide organique vers ledit insert absorbant de recueil (5),
**caractérisée par le fait que** ladite couche lavable comporte, entre ladite enveloppe extérieure (4) et ledit insert (5), un voile intermédiaire (12) réalisé dans un matériau perméable et qui présente, du côté dudit insert absorbant (5), une surface déperlante (12B) conçue apte à faire rouler ledit fluide vers ledit insert absorbant (5).

2. Couche (1) selon la revendication 1, **caractérisée par le fait que** l'enveloppe extérieure (4) comporte sur sa face extérieure (4A) un tissu (7) qui est contrecollé avec une membrane étanche (10) sur sa face intérieure (4B), ladite membrane étanche (10) venant en contact avec le voile intermédiaire déperlant (12).

3. Couche (1) selon l'une des revendications précédentes, **caractérisée par le fait que** le voile intermédiaire déperlant (12) est extensible et comporte de 15 à 25% d'élasthanne.

4. Couche (1) selon l'une des revendications précédentes, **caractérisée par le fait que** le voile intermédiaire déperlant (12) est extensible et comporte du « Lycra » ou est constitué entièrement de « Lycra »

5. Couche (1) selon l'une des revendications précédentes, **caractérisée par le fait que** le voile intérieur de transfert est réalisé en tissu micropolaire ou en suédine ou en textile à base de bambou ou en bourrette de soie (6), et est fixé de façon permanente à l'insert insert absorbant de recueil (5), constituant un ensemble amovible conçu apte à être fixé sur la surface intérieure déperlante (12B) du voile intermédiaire déperlant (12) par des moyens de fixation rapide (11) et des moyens de fixation rapide complémentaires (11C).

6. Couche (1) selon l'une des revendications précédentes, **caractérisée par le fait que** le voile intermédiaire déperlant (12) est constitué d'un textile dont au moins une des surfaces qui constitue la surface intérieure déperlante (12B), est rendue déperlante par imprégnation d'un bain comportant de la saponine.

7. Couche (1) selon l'une des revendications précédentes, **caractérisée par le fait que** le voile intermédiaire déperlant (12) est constitué d'un textile dont au moins une des surfaces qui constitue la surface intérieure déperlante (12B), est rendue déperlante par imprégnation d'un bain comportant au moins une résine fluorée.

8. Couche (1) selon l'une des revendications précédentes, **caractérisée par le fait que** le voile intermédiaire déperlant (12) comporte au moins une ailette (14) extensible saillant de l'enveloppe extérieure (4) et munie de moyens de fixation rapide (11) conçus aptes à coopérer avec des moyens de fixation rapide complémentaires (11C) disposés sur la surface extérieure de l'enveloppe extérieure, pour le maintien en position enveloppée de la couche lavable sur son porteur.

9. Couche (1) selon l'une des revendications précédentes, **caractérisée par le fait que** l'enveloppe extérieure (4) comporte sur sa face extérieure (4A) un tissu (7) qui est contrecollé avec une membrane étanche (10) sur la face intérieure (4B) de ladite enveloppe extérieure (4), ladite membrane étanche (10) venant en contact avec le voile intermédiaire déperlant (12), et ladite membrane (10) est traitée résistante aux rayons ultra-violets, respirante, et résistante à l'humidité.

10. Couche (1) selon l'une des revendications précédentes, **caractérisée par le fait que** l'enveloppe extérieure (4) comporte sur sa face extérieure (4A) un tissu (7) qui est contrecollé avec une membrane étanche (10) sur la face intérieure (4B) de ladite enveloppe extérieure (4), ladite membrane étanche (10) venant en contact avec le voile intermédiaire déperlant (12), et ladite couche (1) comporte des moyens de fixation rapide (11) et des moyens de fixation rapide complémentaires (11C) sous la forme de velours-crochet dont chaque élément est renforcé par une pièce de renfort constituée de la même matière que ledit tissu (7) et cousue sous ce dernier, des pièces de renfort étant aussi fixées sous les éléments des boutons-pression quand ladite couche (1) en comporte.
